# EUROPEAN PATENT SPECIFICATION

(11) **EP 4 015 030 B1**
(45) Date of publication and mention of the grant of the patent: **16.04.2025**
(21) Application number: 21216047.7
(22) Date of filing: 20.12.2021
(51) Int. Cl.: A61M 39/22, F16K 11/085, F16K 35/04

(54) **AN IMPROVED TACTICAL FEEL FOR A MULTILEVEL STOPCOCK**
VERBESSERTES TASTGEFÜHL FÜR EINEN MEHRSTUFIGEN ABSPERRHAHN
SENSATION TACTIQUE AMÉLIORÉE POUR UN ROBINET D'ARRÊT MULTI-NIVEAUX

(30) Priority: 18.12.2020 SE 2030367
(43) Date of publication of application: 22.06.2022
(73) Proprietor: CYTO365 AB, 263 57 Höganäs (SE)
(72) Inventor: TÖRNBLOM, Micael, 263 65 VIKEN (SE)
(74) Representative: AWA Sweden AB

(56) References cited:
- EP-A1- 3 191 168
- EP-B1- 3 191 168
- US-A1- 2017 258 991
- US-A1- 2020 271 232

## Description

### Technical field

The present disclosure refers to a multi-level stopcock valve device with a manipulating device with position indication by enhanced tactical response and the use of such valve device in an intravenous system used for infusion therapy to administer drugs to the patients' vascular system.

### Technical background

There are many types of valve devices typically used within an IV-system, and a common valve device is the stopcock valve device. The stopcock valve device compromises typically of two key components, a valve house and a rotatable valve member, also called a rotor. The rotor is arranged at least partly inside the valve house. The valve house and the rotor share a common rotational axis.

The house may have one or more inlets, also known as an inlet port, which is normally formed as hollow studs extruding radially outwards from the house, positioned at the same rotational plane of the common rotational axis.

The valve normally has one outlet, also known as an outlet port, that extrudes outwards from the valve, by a hollow stud, normally integrated with the valve house. The inlet(s) and the outlet of the valve are normally angularly spaced around the valve's rotational axis on the circumference of the house body.

The rotor is used to divert fluid from normally one the valve's inlet to the valve's outlet, or from more than one of the valve's inlets to the valve's outlet.

The simplest stopcock has only one inlet and one outlet, and is basically just an ON or OFF switch. The rotor has a manipulating device shaped as a valve handle and is normally integrated with the rotor so the user can rotate the rotor to different rotational positions to control fluid of the valve.

The handle typically has two protruding grip vanes, that represents the fluid path of the valve. When the grip vanes are aligned with the inlet and outlet there is a fluid path from the inlet to the outlet, and when the grip vanes is rotated transverse to the inlet and outlet there is no fluid path between the inlet and the outlet of the valve.

One other common medical stopcock is the three-way stopcock, with two inlets and one outlet, where the three-way refers to the number of fluid paths possible in different rotor positions, where any of the two inlets are in fluid communication with the outlet or where two inlets are in fluid communication with each other. Three-way stopcocks may have a stop to hinder the rotor from being rotated to an all open position.

The four-way stopcock also has two inlets and one outlet and the same fluid pathways as with the three way stopcock, but this type of valve also has a fourth position called an all open position where the two inlets are in fluid communication with the outlet.

Furthermore, some stopcocks may have a closed off position where no fluid communication is between any of the inlets and not to the outlet.

The rotor of the above mentioned valves has typically at least a through hole transverse and intersects the rotational axis.

In some stopcocks the channel is formed in the intersection between the rotor and valve house, transverse the rotational axis but not intersecting the rotational axis.

The different fluid communication positions mentioned above are achieved by positioning the rotor around the rotational axis and typically in the same level at a rotational plane transverse of the common rotational axis.

The three-way or four-way stopcock rotor has normally one or more grip vanes that protrudes radially outwards form the rotational axis to provide a good grip for the users' fingers, but also to indicate the fluid flow paths of the valve.

There are normally two types of grip vane philosophy, ON -handle or OFF -handle.

The ON -handle design with a three-way or four-way stopcock normally has three grip vanes, where each grip vane that is co-aligned with the inlets, are in fluid communication with either the outlet or with each other.

The OFF -handle design, normally has only one grip vane and it indicates, when the handle is co-aligned with an inlet, that this inlet is closed off from fluid communication.

The handle grip vane(s) are thereby a representation of the fluid flow within the rotor.

There are some stopcocks where the inlets and outlet are not located at the same rotational plane in relation to the rotational axis. Typically, these valves achieve an administration sequence where only one of the inlets that are located at the same level at the rotational plane of the common rotational axis, is in fluid communication with the outlet, and where there is no fluid path possible between the valve's inlets that are located at the same rotational plane of the common rotational axis. These types of valves may have an angular position between each inlet that is called a flushing position where a neutral flushing fluid is in fluid communication with the outlet but not to any of the other inlets.

These type of multi-level stopcocks normally has a handle integrated with the rotor with one grip vane extending radially outwards from the rotational axis. The protruding grip vane is normally an ON -handle philosophy. When the protruding grip vane is co-linear and over the radially extending inlet, it means that this inlet is in fluid communication with the outlet. The rotor of a multi-level valve has not a through hole transverse the rotational axis since it shall communicate with only one of the inlets in the same plane with the outlet, and close fluid communication from other inlets in the same plane to the outlet. Instead the rotor has a two-part channel, where first part is a radially extending channel from the rotational axis to be moved into and out of alignment with one of the inlets in the same rotational plane, and a second part extending co-axial with the rotational axis that is always aligned with the stopcock outlet.

Single level stopcocks with one outlet connected to the patient via an infusion tube are often limited to two inlets and one outlet. Since the inlets shall be in fluid communication with the outlet, the channel of the rotor must be transverse. This also results in a typical back and forward movement of the three-way and four-way stopcock.

In opposite a multi-level stopcock can have one part of the channel of the rotor always in connection with the valve's outlet, whereas the first part of the channel moves in and out of alignment with an inlet.

The simple structure of the channel in the rotor of the multi-level stopcock has a potential to use many more inlets than a single level stopcock. With more inlets angular displaced, the angular spacing is less. A single-level stopcock may usually have 90 or 180 degree spacing between inlets, a multi-level may have 60 degree.

In between each inlet there may be a flushing position, which then makes spacing of each functional position to only 30 degree.

Tactical feel enhances the user feedback when the user can feel it has reached a functional position. Exemplary valves for administering drug fluids are known from EP 3 191 168 and US 2017/258991.

### Summary

It is an object of the disclosure to provide an enhanced tactical response for a multi-level stopcock with sequential administration of a plurality of drug fluids with an efficient flushing of the valve between each drug fluid administration by rotating the handle and where such enhanced tactical response comprises at least two functional positions, drug position and flushing position.

In contrast to single level stopcocks with a through hole transverse the rotational axis, the multi-level stopcocks have a two-part channel where first part can be aligned with one of respective multiple drug inlet while the second part is always connected to the valve outlet.

Since the multiple flushing positions are consecutive after each drug position when turning the rotor in one rotational direction only there is a need of multiple flushing positions to be used. Multiple flushing position and multiple drug positions require a less angular separation compared to traditional three-way and four-ways stopcocks and thereby a more distinct and more exact tactical response is needed. The multiple positions require a more durable interaction without tearing of any of the materials used.

Another object of the disclosure is to secure the rotor axially in place with an enhanced locking mechanism whilst enabling ease of assembly.

In some embodiments the valve may present the following:
- An enhanced tactile response for a multi-level stopcock, with a rotor having an outlet co-axial with rotational axis and where the stopcock has multiple flushing positions where each flushing position is located in between the previously used drug position to the next drug position in the sequence.
- The rotor receives drug fluids and a flushing fluid from each drug position and each flushing position by rotation of the valve member around its rotational axis, where each position is enhanced by a tactical response.
- The rotor has an enhanced locking mechanism that makes the rotor easy to assemble in axial direction into the stopcock house but where it is harder to dissemble without this securement device.
- A rotor with multiple interaction pairs.

It is known that the sealing interaction between the house and rotor must be maintained and a tactical response should not impair the sealing, and thereby it is preferred to have force exerted radially inwards towards the rotational axis from the house outside. However, in contrary, the collar of this inventive concept is deflecting radially outwards. New directives to avoid misconnection of the commonly used Luer connector as stated in the ISO80369 ISO standard requires that the material of a part with a Luer connector shall have E-modulus over 700 MPa. Meaning that the previous semi-rigid approved material of a stopcock house now needs to be more rigid. Thereby the difference between a typically softer material of a stopcock rotor with a collar and a stopcock house with the new requirements are even greater.

With the new requirements it is likely that deflection of the softer collar radially outwards is the most durable design. The opposite would mean that the soft material that encloses the hard material would need to be significantly larger in size in order to deflect the hard house material inwards. This solution would not be beneficial since it would likely result in shear forces where the soft material would tear more. Another possibility would be to have a very thin design of the harder house material, but it would then probably be too thin and have risk of breaking.

To further improve the torque required to turn the rotor, more interactive pairs may be used in the collar.

Another object of the disclosure is to provide an enhanced axial locking mechanism of the rotor in an axial position inside the valve house. The rotor and house are axially fixed by an annular protruding ring of the rotor that fits into the annular recess of the top part of the house.

The annular collar may act as an anvil when the rotor is moved in an axially upwards direction, due to the fact that the rotor collar encloses the top part of the house and the resulting radially outward force from the top part of the house is counter acted by the radially inward force of the enclosing collar. The axial locking force increases if the distance from the top part of the house is increased to where the annular undercut is, and the distance of the collar and preferably the collar should reach below the axial position of the undercut.

When mounting the rotor axially in place inside the house, the rotor collar will first enclose and reach below the top part of the house, before the top part of the house deflects outwards due to the rotor annular ring. When the rotor is pressed in place the top part of the relatively hard house compared to the collar will force the collar to deflect outwards but not enough to plastically deform the collar.

According to a first aspect there is provided a valve for administering drug fluid as defined in appended independent claim 1. Further aspects are defined by the appended dependent claims.

### Definitions

Rotational position should be interpreted as any angular position of the rotor around the rotational axis.

Functional position should be interpreted as a rotational position where there is a tactile response.

The inlets and outlet of the valve may interpreted as the opposite.

Further objects and advantages of the present disclosure will be obvious to a person skilled in the art reading the detailed description given below describing different embodiments.

### Brief description of the drawings

The valve of the disclosure will now be described in detail with reference to the schematic drawings.
Fig. 1 disclose one embodiment assembly, in a perspective view
Fig. 2A disclose the stopcock house, in a perspective view
Fig. 2B disclose the stopcock house, in a side view
Fig. 3 disclose the rotor, in a perspective view
Fig. 4A disclose the rotor, in a front view
Fig. 4B disclose the rotor, in a section view transverse section A-A in Fig. 4A

### Detailed description

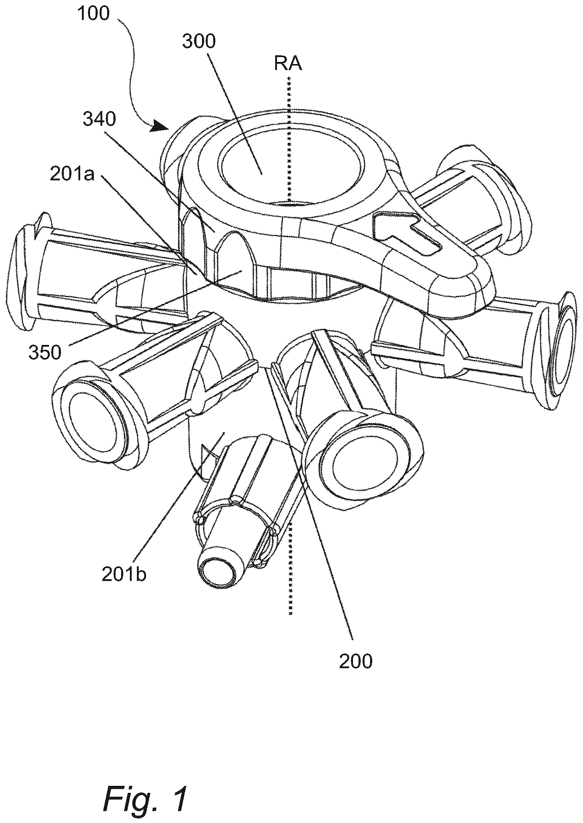

Fig. 1 show a multi-level stopcock valve 100, with a house 200 and a rotor 300. The rotor is rotatable arranged partly inside the house, where the rotor and house share a common rotational axis RA. The valve has a manipulating device 340 that is in this image shown as integrated as a part of the rotor. An annular collar 350 is shown axially below the manipulating device. The house has a top part 201a which is axially closer to the manipulating device than the bottom part of the house 201b. The annular collar 350 is enclosing at least partly the top part of the house 201a. In this embodiment, the house bottom part 201b has a closed bottom, meaning that there is no through going hole in an axially direction of the house.

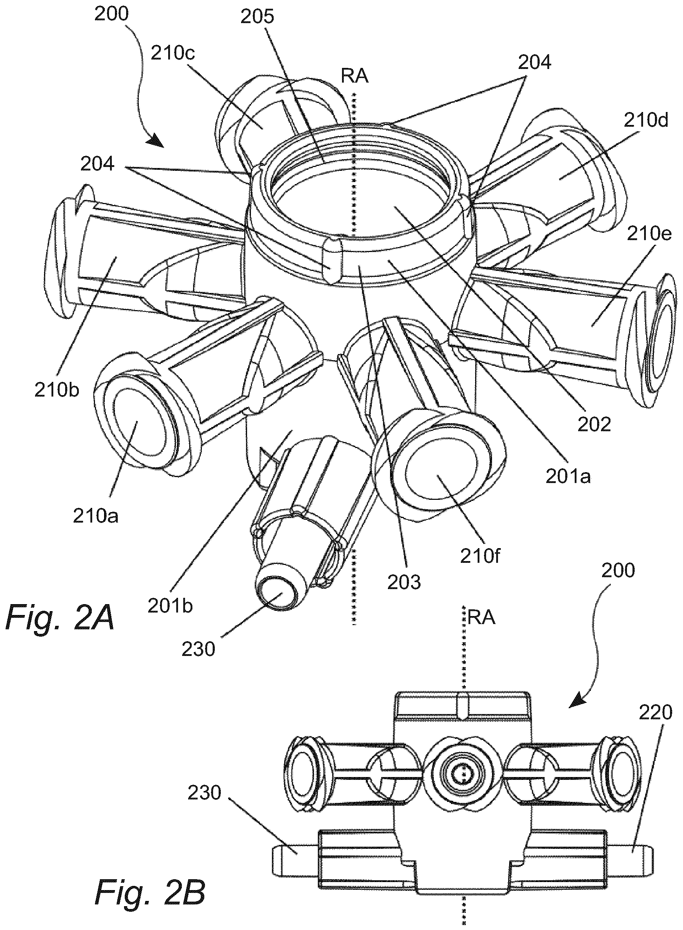

Now turning to Fig. 2A that is showing the house 200 with the rotational axis RA. The top part 201a has interaction means made by protruding notches 204, that are angularly spaced around rotational axis at 90 degree apart. The inner circumferential surface 202 has a circumference smaller than the circumference of the outer circumferential surface 203. The rotor is axially locked in place by a retainer undercut 205 formed at the top part 201a of the house. The house has one or more inlets 210a-f, for received drug fluids. All inlets 210a-f, are arranged at the same plane transverse the rotational axis. As best seen in Fig. 2B, there is another inlet 220 located axially spaced at another level at a plane transverse the rotational axis then the inlets 201a-f. In this type of embodiment, it is not possible to have fluid communication between each inlet at the same level. The fluid exits the valve via an outlet 230.

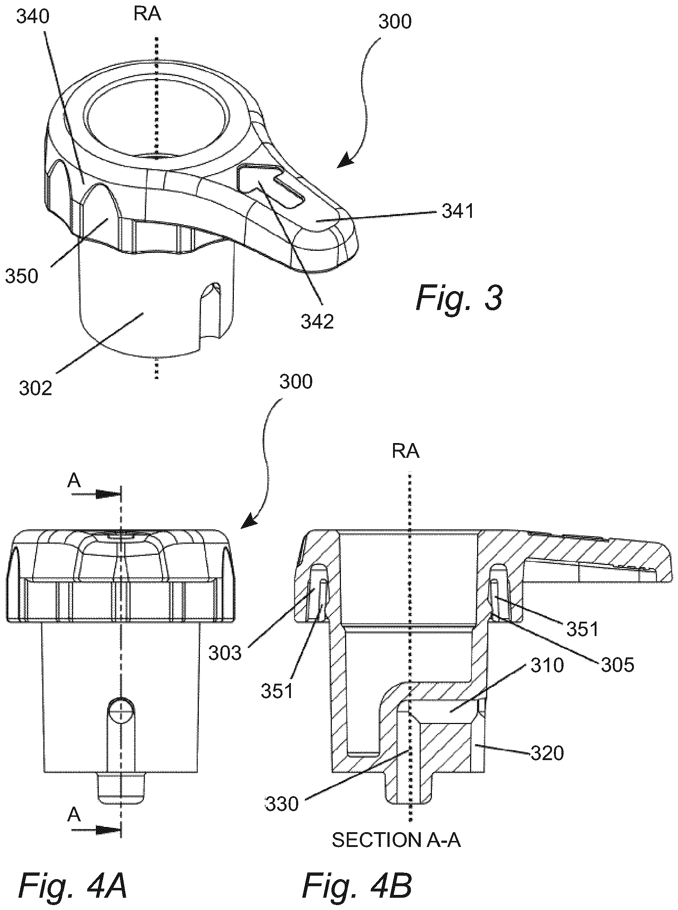

Now turning to Fig. 3, 4A and 4B, that is showing the rotor 300, with the rotational axis RA. The rotor has an outer surface 302 that is sealingly engaging the inner circumferential surface of the house 202 shown in Fig 2.

The rotor is attached to a manipulating device 340 that is in this embodiment shown as integrated with the rotor. The annular collar 350 is axially placed below the manipulating device. There is a protruding grip vane 341 attached to the manipulating device 340 and in this embodiment, it is integrated with the manipulating device. There is an arrow symbol 342 to visually aid the user that this handle is an ON handle and showing the intended flow direction. The rotor has in this embodiment two inlets, where the inlet channel 310 is extending radially from the inner rotational axis to the outer circumferential surface 302. The inlet channel 310 can receive fluid from the respective one of the fluid inlets 210a-f when the rotor is positioned to the position where the radially extending inlet channel 310 is rotationally aligned with an inlet. The open recess inlet 320 can in this embodiment receive fluid from the inlet 220 shown in FIG 2B. in any rotational positions of the rotor. The centrally placed outlet of the rotor 330 is co-axial with the rotational axis and is always in fluid communication with the outlet 230 shown in FIG 2A,2B.

The axially recessed grooves 351 are formed on the circumferential surface 303 of the annular collar 350. The number of recessed grooves is in this embodiment are 12 and they are spaced 30 degrees apart that will give a tactile response every 30 degree. The number of grooves can vary depending on how many functional positions there are on the valve that will be assigned with a tactical response, typically but not limited to between 4-12 recess grooves and preferably but not limited to equally angularly spaced apart. The circumferential surface 303 has a circumference larger than the circumference of the outer circumferential surface 203. The collar 350 extending in an axially direction and partly encloses the top part 201a of the house 200 so that the grooves 351 and the protruding notches 204 can engage with each other in each intended rotational position. In other words, the grooves 351 and the protruding notches 204 can engage with each other in each functional position. The collar will deflect radially outwards due to its softer material compared to the housing material and due to the collar relatively flexible and thin design. The annular extending protruding ring 305 is locking together with the annular recessed undercut 205 of the top part 201a of the house.

## Claims

1. A valve for administering drug fluid, comprising:
- a rotational axis (RA),
- at least one inlet (210a-f; 220) for receiving a drug fluid,
- a valve outlet (230),
- a house body (200),
- a rotor (300), and
- a manipulating device (340) configured to manipulate said rotor to be rotated about said rotational axis,
wherein said house body (200) having:
- an inner cavity,
- an inner circumferential sealing surface (202),
- a top part (201a) and a bottom part (201b) which are mutually spaced along the rotational axis (RA), wherein the bottom part (201b) is located, more distally from the manipulating device along the rotational axis than the top part, and wherein said top part has an outer circumferential surface with a circumference larger than said inner circumferential surface, and protruding ribs on said outer circumferential surface,
wherein said rotor having:
- a circumferential sealing surface,
- an annular collar (350) which has a circumference larger than said outer circumferential surface of said house top part, and which at least partly encloses said top part of said house body, and said annular collar having axial recess grooves (351) arranged to engage with said protruding ribs of the house body,
- an annular protruding ring (305), and
- a passageway (310, 330) presenting an inlet arranged in the circumferential sealing surface of the rotor, and an outlet arranged co-axially with said rotational axis and always in fluid communication with said valve outlet (230),
wherein said rotor is rotatable about said rotational axis and arranged at least partly within said inner cavity of said house body, said circumferential sealing surface of said rotor being in sealing engagement with said inner circumferential sealing surface of said house, and said rotor being configured to be rotated into any selected one of a plurality of rotational positions defined by said recess grooves engaging with said protruding ribs, and
wherein the top part of the house body includes an annular recess (205) and the annular collar extends axially past the protruding annular ring (305) of said rotor to provide a locking mechanism enabled by a radially outward force from the top part of the house body being counter acted by a radially inward force from the enclosing collar.

2. Valve according to claim 1, wherein the at least one inlet (210a-f; 220) is a plurality of inlets (210a-f; 220) which includes a first inlet which is located in a plane transverse to the rotational axis and axially spaced from the other inlets (201a-f) of the plurality of inlets along the rotational axis, and wherein there is fluid communication between the first inlet and the outlet in all rotational positions.

3. Valve according to claim 1 or 2, wherein the protruding ribs are positioned on the outer circumferential surface and the axial recesses are positioned on the annular collar such that the tactile response between each functional position of the valve is less than 45 degrees.

4. Valve according to any of the preceding claims, wherein the number of axial recess grooves (351) are between 4 and 12.

5. Valve according to any of the preceding claims, wherein the axial recess grooves (351) are equally angularly spaced apart.

## Patentansprüche

1. Ventil zum Verabreichen eines Arzneimittelfluids, umfassend:
- eine Rotationsachse (RA),
- mindestens einen Einlass (210a-f; 220) zur Aufnahme eines Arzneimittelfluids,
- einen Ventilauslass (230),
- einen Gehäusekörper (200),
- einen Rotor (300), und
- eine Manipulationsvorrichtung (340), die dazu konfiguriert ist, den Rotor so zu manipulieren, dass er um die Rotationsachse rotiert,
wobei der Gehäusekörper (200) aufweist:
- einen inneren Hohlraum,
- eine innere Umfangsabdichtoberfläche (202),
- einen oberen Teil (201a) und einen unteren Teil (201b), die entlang der Rotationsachse (RA) voneinander beabstandet sind, wobei sich der untere Teil (201b) entlang der Rotationsachse weiter distal von der Manipulationsvorrichtung befindet als der obere Teil und wobei der obere Teil eine äußere Umfangsoberfläche mit einem Umfang aufweist, der größer ist als die innere Umfangsoberfläche, und hervorstehende Rippen auf der äußeren Umfangsoberfläche,
wobei der Rotor aufweist:
- eine Umfangsabdichtoberfläche,
- einen ringförmigen Kragen (350), der einen größeren Umfang als die äußere Umfangsoberfläche des Oberteils des Gehäuses aufweist und der den oberen Teil des Hauskörpers mindestens teilweise umschließt, wobei der ringförmige Kragen axiale Aussparungsnuten (351) aufweist, die so angeordnet sind, dass sie mit den hervorstehenden Rippen des Gehäusekörpers in Eingriff kommen,
- einen ringförmigen hervorstehenden Ring (305) und
- einen Durchgang (310, 330), der einen in der Umfangsabdichtoberfläche des Rotors angeordneten Einlass und einen koaxial zur Rotationsachse angeordneten und stets in Fluidverbindung mit dem Ventilauslass (230) stehenden Auslass aufweist,
wobei der Rotor um die Rotationsachse rotierbar ist und mindestens teilweise innerhalb des inneren Hohlraums des Gehäusekörpers angeordnet ist, wobei die Umfangsabdichtoberfläche des Rotors in abdichtendem Eingriff mit der inneren Umfangsabdichtoberfläche des Gehäuses steht und der Rotor so konfiguriert ist, dass er in eine beliebige ausgewählte einer Vielzahl von Rotationspositionen rotiert werden kann, die durch die in Eingriff mit den hervorstehenden Rippen stehenden Aussparungsnuten definiert sind, und wobei der obere Teil des Gehäusekörpers eine ringförmige Aussparung (205) aufweist und der ringförmige Kragen axial über den hervorstehenden ringförmigen Ring (305) des Rotors hinausragt, um einen Verriegelungsmechanismus bereitzustellen, der durch eine radial nach außen gerichtete Kraft vom oberen Teil des Gehäusekörpers aktiviert wird, der eine radial nach innen gerichtete Kraft vom umschließenden Kragen entgegenwirkt.

2. Ventil nach Anspruch 1, wobei der mindestens eine Einlass (210a-f; 220) eine Vielzahl von Einlässen (210a-f; 220) ist, die einen ersten Einlass einschließt, der sich in einer Ebene quer zur Rotationsachse befindet und axial von den anderen Einlässen (201a-f) der Vielzahl von Einlässen entlang der Rotationsachse beabstandet ist, und wobei in allen Rotationspositionen eine Fluidverbindung zwischen dem ersten Einlass und dem Auslass besteht.

3. Ventil nach Anspruch 1 oder 2, wobei die hervorstehenden Rippen auf der äußeren Umfangsoberfläche und die axialen Aussparungen auf dem ringförmigen Kragen so angeordnet sind, dass die taktile Reaktion zwischen jeder Funktionsposition des Ventils weniger als 45 Grad beträgt.

4. Ventil nach einem der vorhergehenden Ansprüche, wobei die Anzahl der axialen Aussparungsnuten (351) zwischen 4 und 12 liegt.

5. Ventil nach einem der vorhergehenden Ansprüche, wobei die axialen Aussparungsnuten (351) im gleichen Winkelabstand angeordnet sind.

## Revendications

1. Valve destinée à l'administration d'un fluide médicamenteux, comprenant :
un axe de rotation (RA),
au moins une entrée (210a-f ; 220) permettant de recevoir un fluide médicamenteux,
une sortie de valve (230),
un corps de boitier (200),
un rotor (300), et
un dispositif de manipulation (340) configuré pour manipuler ledit rotor afin de le faire tourner autour dudit axe de rotation,
dans lequel ledit corps de boitier (200) comporte :
- une cavité intérieure,
- une surface d'étanchéité circonférentielle intérieure (202),
- une partie supérieure (201a) et une partie inférieure (201b) qui sont mutuellement espacées le long de l'axe de rotation (RA), dans laquelle la partie inférieure (201b) est située plus loin du dispositif de manipulation le long de l'axe de rotation que la partie supérieure, et dans laquelle ladite partie supérieure a une surface circonférentielle extérieure avec une circonférence plus grande que ladite surface circonférentielle intérieure, et des nervures en saillie sur ladite surface circonférentielle extérieure,
dans laquelle ledit rotor comporte :
- une surface d'étanchéité circonférentielle,
- un collier annulaire (350) qui a une circonférence plus grande que ladite surface circonférentielle extérieure de ladite partie supérieure du boitier, et qui entoure au moins partiellement ladite partie supérieure dudit corps de boitier, et ledit collier annulaire ayant des rainures d'évidement axiales (351) conçues pour s'engager dans lesdites nervures en saillie du corps de boitier,
- une bague annulaire saillante (305), et
- un passage (310, 330) présentant une entrée disposée dans la surface d'étanchéité circonférentielle du rotor, et une sortie disposée de manière coaxiale avec ledit axe de rotation et toujours en communication fluidique avec ladite sortie de soupape (230),
dans laquelle ledit rotor peut tourner autour dudit axe de rotation et est disposé au moins en partie à l'intérieur de ladite cavité intérieure dudit corps de boîtier, ladite surface d'étanchéité circonférentielle dudit rotor étant en prise étanche avec ladite surface d'étanchéité circonférentielle intérieure dudit boîtier, et ledit rotor étant configuré pour être mis en rotation dans l'une quelconque d'une pluralité de positions de rotation définies par lesdites rainures d'évidement venant en prise avec lesdites nervures en saillie, et
dans laquelle la partie supérieure du corps du boîtier comprend un renfoncement annulaire (205) et le collier annulaire s'étend de manière axiale au-delà de la bague annulaire saillante (305) dudit rotor pour fournir un mécanisme de verrouillage activé par une force radiale vers l'extérieur de la partie supérieure du corps du boîtier qui est contrecarrée par une force radiale vers l'intérieur du collier d'enfermement.

2. Valve selon la revendication 1, dans laquelle l'au moins une entrée (210a-f ; 220) est une pluralité d'entrées (210a-f ; 220) qui comprend une première entrée qui est située dans un plan transversal à l'axe de rotation et espacée de manière axiale des autres entrées (201a-f) de la pluralité d'entrées le long de l'axe de rotation, et dans laquelle il existe une communication fluidique entre la première entrée et l'orifice de sortie dans toutes les positions de rotation.

3. Valve selon la revendication 1 ou la revendication 2, dans laquelle les nervures saillantes sont positionnées sur la surface circonférentielle extérieure et les renfoncements axiaux sont positionnés sur le collier annulaire de telle sorte que la réponse tactile entre chaque position fonctionnelle de la valve est inférieure à 45 degrés.

4. Valve selon l'une quelconque des revendications précédentes, dans laquelle le nombre de rainures axiales (351) est compris entre 4 et 12.

5. Valve selon l'une quelconque des revendications précédentes, dans laquelle les rainures axiales (351) sont également espacées angulairement.
